# EUROPÄISCHE PATENTSCHRIFT

(11) **EP 0 396 561 B1**
(45) Veröffentlichungstag und Bekanntmachung des Hinweises auf die Patenterteilung: **02.11.1994**
(21) Anmeldenummer: 88909740.8
(22) Anmeldetag: 14.11.1988
(51) Int. Cl.: A61B 17/12, A44B 11/25

(54) **VORRICHTUNG ZUR BLUTSTAUUNG IN KÖRPERTEILEN**
DEVICE FOR LIGATURING PARTS OF THE BODY
DISPOSITIF HEMOSTATIQUE APPLICABLE A DES PARTIES DU CORPS

(30) Priorität: 17.11.1987 DE 3738903
(43) Veröffentlichungstag der Anmeldung: 14.11.1990
(73) Patentinhaber: PRÄMETA Gesellschaft für Präzisionsmetall- und Kunststofferzeugnisse mbH & Co. KG, 51107 Köln (DE)
(72) Erfinder: Sturm, Martina Elisabeth, D-51377Leverkusen (DE)
(74) Vertreter: Dallmeyer, Georg, Dipl.-Ing.
(86) Internationale Anmeldenummer: DE8800710
(87) Internationale Veröffentlichungsnummer: WO8904637

(56) Entgegenhaltungen:
- WO-A-89/04636
- DE-A- 3 538 583
- FR-A- 2 501 484
- US-A- 3 349 449

## Beschreibung

Die Erfindung bezieht sich auf eine Vorrichtung zur Blutstauung in Körperteilen nach dem Oberbegriff des Patentanspruchs 1.

Bei dieser aus der DE-OS 35 38 583 vorbekannten Vorrichtung verläuft die Grundfläche bis zur der Schlinge zugewandten Vorderkante des Klemmteils eben. Der eine Schlinge bildende Gurtteil wird an der vordersten Kante dieser Grundfläche in den Verlauf der Grundfläche abgeknickt und im weiteren Verlauf der Grundfläche durch den Klemmbereich klemmend festgelegt.

Bei der praktischen Verwendung der Vorrichtung wird die Schlinge um ein Körperteil, beispielsweise einen Arm, gelegt und das durch das Klemmteil hindurchreichende Gurtende solange gezogen, bis die Schlinge ausreichend fest den Arm umspannt. Bei diesem Vorgang wird die Schlinge zusammengezogen, ein Teilstück des Gurtes läuft über die beschriebene Vorderkante in das Klemmteil ein, dementsprechend wird das hinten aus dem Klemmteil überstehende Gurtende länger. Beim Zusammenziehen der Schlinge und Einlauf des Gurtes in den Klemmteil wird jedoch die an der Schlingeninnenseite liegende Haut reibschlüssig mitgenommen, die Haut bildet eine Falte, die beim weiteren Anziehen der Schlinge in die Gurtschnalle hineingezogen und dort zunehmend stärker festgeklemmt wird. Dies verursacht Schmerzen.

Die Erfindung hat sich zur Aufgabe gemacht, die Vorrichtung der eingangs genannten Art dahingehend zu verbessern, daß ein Einziehen von Haut in die Gurtschnalle nur in einem Maße erfolgen kann, das nicht Schmerzen auslöst.

Diese Aufgabe wird gelöst durch die Vorrichtung nach dem Patentanspruch 1.

Durch die Querrippe wird der Gurt zunächst höher als für das Einlaufen in die Grundfläche erforderlich angehoben, er verläuft anschließend auf die Grundfläche zu und liegt zumindest im Klemmbereich auf dieser auf. Der Gurt wird also insgesamt stärker als beim Stand der Technik abgewinkelt. Dies hat zur Folge, daß reibschlüssig mitgenommene Haut hinter der Querrippe und damit im Inneren der Gurtschnalle nicht mehr am Gurt anliegt, die Querrippe bewirkt eine Trennung zwischen Gurt und von diesem reibschlüssig mitgenommener Haut.. Man kann hier auch von einer Art Abrißkante sprechen. Dadurch kommt es nicht zu einer engen Einpressung der Haut und auch zu keinen Schmerzen. Die Haut wird zwar nachwievor vom Gurt mitgenommen, aber nicht quer zum Gurt in eine Engstelle hineingezogen, wo ein Zwicken oder Klemmen auftreten könnte.

In einer Weiterbildung hat die Querrippe eine Schrägfläche für die Gurtauflage, deren Verlauf im wesentlichen der Richtung des einlaufenden Teilbereichs der Schlinge entspricht. Hierdurch erfolgt die Abknickung des Gurtes nicht unmittelbar an der Stirnfläche, sondern einige Millimeter, beispielsweise drei bis fünf Millimeter, dahinter.

In einer weiteren Verbesserung verläuft die Schrägfläche im Winkel von 30 bis 60 Grad, vorzugsweise im Winkel von 45 Grad zur Grundfläche. Hierdurch wird der erwünschte, relativ große Abknickwinkel des Gurtes erreicht. Die Schrägfläche definiert den Einlaufwinkel, hinter der Schrägfläche und der Querrippe wird der Gurt wieder in Gegenrichtung zur Grundfläche hin abgewinkelt. Hierdurch ergibt sich insgesamt eine Abwinklung, die mindestens 50 Grad (bezogen auf die nicht abgeknickte Richtung) beträgt.

In einer weiteren Verbesserung ist die Oberkante der Querrippe gegenüber einer der Schlinge zugewandten Stirnfläche des Klemmteils versetzt, vorzugsweise mindestens um die Gurtbreite versetzt. Auch hierdurch wird erreicht, daß das Abknicken des Gurtes innerhalb des Gurtschlosses erfolgt.

Weitere Vorteile und Merkmale der Erfindung ergeben sich aus den übrigen Ansprüchen sowie der nun folgenden Beschreibung eines Ausführungsbeispiels, das unter Bezugnahme auf die Zeichnung näher erläutert wird. In dieser zeigen:
- Fig. 1: zeigt eine Ausführungsform der Vorrichtung in Ansicht,
- Fig. 2: zeigt die Vorrichtung aus Ansicht 1 im Schnitt,
- Fig. 3: stellt die Vorrichtung in einer Draufsicht dar,
- Fig. 4: ist eine stirnseitige Ansicht mit geschnittenem Gurt,
- Fig. 5: eine Draufsicht entsprechend Fig. 3, jedoch nur auf das Klemmteil 1, also bei abgenommenem Löseteil 2 und
- Fig. 6: ein Wirkschema der Hebel und der Kraftverteilung bei einer Darstellung entsprechend Fig. 1.

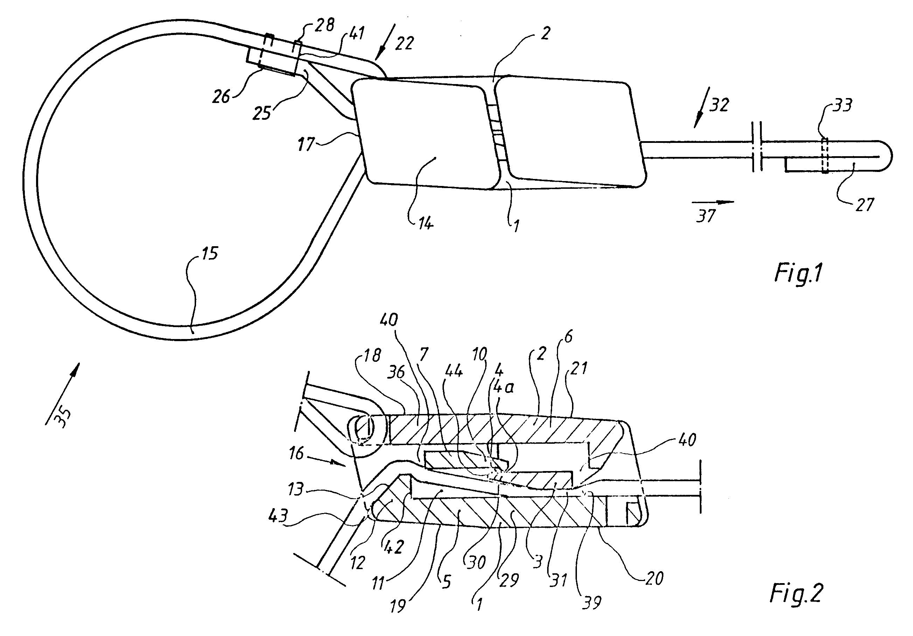

Die Darstellungen der Fig. 1 bis 5 zeigen die Vorrichtung in gespannter Gebrauchsposition.

Die Vorrichtung besteht aus einem, mit einem Teil seiner Gesamtlänge die Schlinge 35 bildenden Gurt 15, dem Klemmteil 1 und dem Löseteil 2. Klemmteil 1 und Löseteil 2 sind geometrisch gleiche Teile (baugleiche Teile) und können durch Längsverschiebung (in Längsrichtung des Gurtes 15) miteinander verhakt und in Gegenrichtung hierzu gelöst werden. Auf einem im wesentlichen rechteckförmigen Grundkörper 29 ist eine brückenartige Konstruktion angeordnet, die jeweils aus zwei haugleichen Seitenwangen 14 und einem diese verbindenen Brückenteil 7 bestehen. Letzteres hat auf einer Seite einen dachförmigen, angeschrägten Überstand 10. Im ausgestreckten Zustand des Gurtes 15, also bei gelöster Schlinge 35, befindet sich das Löseteil 2 an einem Ende, das Klemmteil im Verlauf des Gurtes 15. In dieser Anordnung stehen die beiden Teile 1, 2 in Längsrichtung des Gurtes 15 versetzt zueinander. Ausgehend von diesem Zustand wird zur Bildung der Schlinge 35 ein Teil um 180 Grad zu einer quer zur Gurtebene liegenden Achse bewegt, wodurch die in den Figuren 1 bis 4 und 6 gezeigte Anordnung der beiden Teile 1, 2 erhalten wird, bei dem der dachförmige Überstand 10 des Löseteils 2 unter den dachförmigen Überstand 30 des Klemmteils 1 hakt und gleichzeitig an dieser Stelle ein Schwenkgelenk gebildet wird.

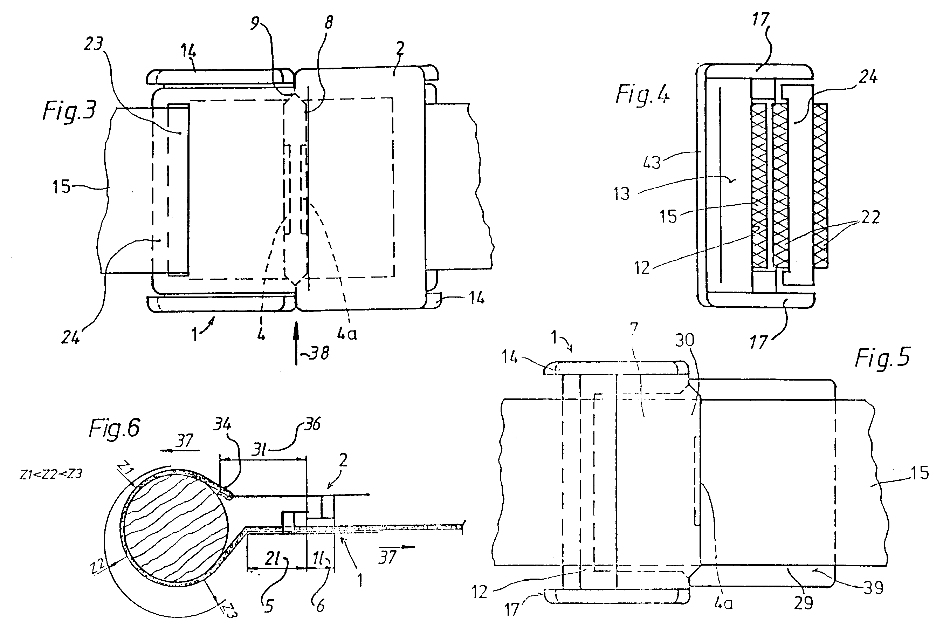

Liegt in der Schlinge 35 kein Zug vor, befindet sich also die Gurtschnalle nicht in gespannter Gebrauchsposition, so ist die lichte Öffnung 31 des Klemmbereichs 3 gerade so ausreichend groß, daß der Gurt 15 in seiner Längsrichtung verschiebbar ist. Sobald jedoch, wie in Figur 6 angegeben, in der Schlinge 6 eine Zugspannung besteht und dadurch die der Schlinge 35 zugewandten Frontbereiche der beiden Teile 1, 2 voneinander wegbewegt werden, wird die gespannte Gebrauchsposition erhalten, wie sie in den Figuren dargestellt ist. Dabei wird die lichte Öffnung 31 soweit verringert, daß der im Klemmbereich 3 befindliche Gurt 15 festgeklemmt ist. Der Gurt 15 ist in dem Klemmteil 1 durch eine Kammer 11 geführt, das lose Gurtteil 32 ist zur Sicherung gegen ungewolltes Herausziehen durch Umschlagen des Gurtmaterials (Überlappung 27) verdickt und durch eine Verklammerung 33 befestigt.

Das andere, fest mit dem Löseteil 2 verbundene Gurtende 22, ist durch Umschlingen des Steges 24 und längsangeordnete Verklammerung 26 des überlappenden Gurtteils 25 unverlierbar verbunden. Das Gurtmaterial ist längselastisch und zugleich querelastisch.

Der Gurt 15 wird in Form einer Schlinge 35 um das abzubindende Körperteil gelegt. Das Löseteil 2 wird mit dem Klemmteil 1 durch Längsverschieben verhakt. Der Gurt 15 wird durch Längszug am freien Gurtende gespannt. Dadurch wird das Klemmteil 1 im Klemmbereich 3 gegen den Gurt gedrückt. Die entstehende Gurtspannung Z steigt am Schlingenumfang von der Anschlaufung 34 des Löseteils 2 zum Klemmteil 1 hin an (Z1, Z2, Z3), so daß über den langen Hebelarm 5 des Klemmteils 1 und den gemeinsamen Drehpunkt 4 der Gurt 15 im Klemmbereich 3 gegen Längsverschiebung durch Klemmung gesichert wird.

Der Stauzustand wird durch die vorstehenden Flächen 17 auch im Gurteinzugbereich der Vorrichtung erreicht.

Die Gurtzugkraft "Z" baut sich im Verlauf der Umschlingung des Körperteils ab, so daß dem Lösen des Löseteils 2 nur die Restzugkraft "Z1" entgegenwirkt.

Die Lösehebellänge 36 bedingt zur Aufhebung der Klemmwirkung am Klemmbereich 3 einen großen Hub am vorderen Löseteil 2 in Abwärtsrichtung zum Klemmteil 1 hin und ermöglicht ein sanftes Entstauen. Der sanfte Entstauvorgang wird zusätzlich durch eine stirnseitige Gurtumlenkung an einer Querrippe 12 und der dabei entstehenden Gurtreibung verstärkt.

Das Löseteil 2 ist nach dem Aufheben der Gurtzugkraft durch leichtes Zurückziehen des dachförmigen Überstandes 10 des Löseteils 2 aus der Kammer 11 des Klemmteils 1 trennbar.

Die Fläche 18 des Löseteils 2 bildet im entspannten Zustand der Vorrichtung mit der Fläche 19 des Klemmteils 1 einen zur Gurtschlinge 35 geneigten Keil zur Unterstützung der Handhabung beim Entstauen, Lösen und Trennen. Die im entspannten Zustand vorliegende Verdickung des Gurtmaterials im Klemmbereich 3 drückt die Teile in die vorgenannte vorteilhafte Position.

Eine Querrippe 12, die sich über die gesamte Gurtbreite erstreckt und rechtwinklig zum Gurt verläuft, befindet sich am vorderen Ende des Grundkörpers 29. Die Querrippe 12 kann beliebig ausgebildet sein. Im gezeigten Ausführungsbeispiel ist sie einstückiges Bestandteil des auch ansonsten einstückigen Klemmteils 1, sie kann aber auch durch eine eingesetzte, gegebenenfalls drehbare Achse dargestellt sein, als Anzahl von nebeneinander quer in Gurtrichtung angeordneten Vorsprüngen ausgeführt sein oder eine ähnliche Ausbildung haben. Entscheidend ist lediglich, daß der Gurt über eine Kante geführt wird, die im gezeigten Ausführungsbeispiel durch die höchste Erhebung der Querrippe 12 ausgebildet ist. Der Grundkörper bildet an seiner oberen, inneren Seite eine Grundfläche 39 auf, die sich praktisch über die gesamte Länge des Grundkörpers erstreckt. Die Querrippe 12 springt von dieser Grundfläche 39 mehrere Millimeter, beispielsweise 2 bis 3 mm, vor, anders ausgedrückt springt sie mindestens um die einfache Gurtdicke, in vorzugsweiser Ausführung mindestens um die zweifache Gurtdicke (also 4 bis 6 mm) gegenüber der Grundfläche 39 vor. Zur Stirnfläche 17 wird die Querrippe 12 durch eine Schrägfläche 13 begrenzt, die im Winkel von 45 Grad zur Grundfläche 39 verläuft. Dadurch auch befindet sich die Oberkante der Querrippe 12 nicht in unmittelbarer Nähe der Stirnfläche 17 - was ausdrücklich nicht ausgeschlossen werden soll - sondern nach innen hin versetzt, im gezeigten Ausführungsbeispiel um ca. 5 mm nach innen versetzt. Die Schrägfläche 13 geht zur vorderen Außenfläche 19 des Klemmteils 1 in einer gerundeten Körperkante 43 über, diese befindet sich geringfügig (wenige zehntel Millimeter) hinter der Stirnfläche 17. Auf der vom Klemmteil 1 abgewandten Seite des Gutes 15 bleibt zwischen Stirnfläche 17 und Oberkante der Querrippe 12 ein Freiraum zum Löseteil 2 bzw. zum Gurtende 22 frei, der breiter ist als die einfache Gurtbreite. Insbesondere läuft er nicht trichterförmig zu, sondern weitet sich hinter der Oberkante und bis zum Beginn der brückenartigen Konstruktion 7 des Klemmteil 1 wieder auf, so daß ein trichterförmiges Einziehen von Haut hier verhindert wird.

Zwischen Querrippe 12 und dem Brückenteil 7 des Klemmteils 1 wird ein Langloch für den Durchlauf des Gurtes ausgebildet, der Gurt durchläuft hinter diesem Langloch eine Kammer 11 und gelangt anschließend unter den Klemmbereich 3, wo er festgelegt wird. Erst dort ist er in Berührung mit der Grundfläche 39.

Eine Kante des Brückenteils, das sich über die gesamte Gurtbreite erstreckt, liegt an der die Innenseite der Schlinge bildenden Fläche des Gurtes an. Wie Fig. 2 zeigt, ist diese Kante so zur Querrippe 12 angeordnet, daß der Gurt an ihr eine leichte Abknickung in Gegenrichtung zu der Abknickung erfährt, die an der Querrippe auftritt. Dadurch erhält der Gurt einen geringfügig S-förmigen Verlauf, wobei jedoch die Abknickung an der Kante des Brückenteils 7 deutlich geringer ist, beispielsweise kleiner als 10 Grad beträgt, während die Abwinklung an der Querrippe mindestens fünfmal, vorzugsweise zehnmal größer ist.

## Patentansprüche

1. Vorrichtung zur Blutstauung in Körperteilen mit einem eine Schlinge (35) bildenden, flachen Gurt (15) aus einem elastischen Material und einer an diesem befestigten Gurtschnalle, die aus einem Klemmteil (1) und einem Löseteil (2) besteht, wobei das Klemmteil einen Grundkörper (29) aufweist, der eine Grundfläche (39) für die Auflage und Führung in Längsrichtung des Gurtes (15) ausbildet und Seitenwangen hat, die erste Verriegelungsmittel für eine lösbare Verbindung von Klemmteil (1) und Löseteil (2) tragen und zwischen denen der Gurt (15) verläuft, und das Löseteil (2) mit einem Gurtende (22) verbunden ist, zweite Verriegelungsmittel für die lösbare Verbindung mit dem Klemmteil (1) hat und an seinem von der Schlinge (35) abgewandten Endbereich einen Klemmbereich (3) aufweist, dessen Abstand von der Grundfläche (39) im zusammengesetzten Zustand der Gurtschnalle im Bereich der Dicke des entspannten Gurtes (15) liegt und im zusammengesetzten Zustand der Gurtschnalle einerseits das Klemmteil (1) mit dem Löseteil (2) über die ersten und zweiten Verriegelungsmittel verbunden ist und andererseits die ersten und zweiten Verriegelungsmittel ein Schwenkgelenk bilden und bei einem Druck auf eine vordere Außenfläche (18) des Löseteils (2), die sich zwischen den ersten und zweiten Verriegelungsmitteln und der Schlinge (35) befindet, der Klemmbereich (3) von der Grundfläche (39) entfernt wird und dadurch die Klemmwirkung aufgehoben wird,
dadurch gekennzeichnet, daß am der Schlinge (35) zugewandten Ende des Klemmteils (1) eine Querrippe (12) von der Grundfläche (39) zum Löseteil (2) hin vorspringt, daß diese Querrippe (12) zumindest um die einfache, Vorzugsweise zumindest die doppelte Gurtdicke gegenüber der Grundfläche (39) vorsteht, so daß der Gurt (15) im Anschluß an die Querrippe (12) auf die Grundfläche (39) zu verläuft.

2. Vorrichtung nach Anspruch 1, dadurch gekennzeichnet, daß die Querrippe (12) eine Schrägfläche (13) für die Gurtauflage ausbildet, deren Verlauf im wesentlichen der Richtung des anliegenden, einlaufenden Teils der Schlinge (35) entspricht.

3. Vorrichtung nach Anspruch 1 oder 2, dadurch gekennzeichnet, daß die Schrägfläche (13) im Winkel von 30 bis 60 Grad, vorzugsweise 45 Grad zur Grundfläche (39) verläuft.

4. Vorrichtung nach einem der Ansprüche 1 bis 3, dadurch gekennzeichnet, daß die Oberkante der Querrippe (12) gegenüber einer der Schlinge (35) zugewandten Stirnfläche (17) des Klemmteils (1) nach innen hin versetzt ist, vorzugsweise mindestens um die Gurtbreite bzw. um 4 bis 6 mm von der Stirnfläche (17) entfernt ist.

5. Vorrichtung nach einem der Ansprüche 1 bis 4, dadurch gekennzeichnet, daß der Gurt an der Querrippe (12) eine Abknickung um mindestens 50 Grad, vorzugsweise um mindestens 60 Grad erfährt.

6. Vorrichtung nach einem der Ansprüche 1 bis 5, dadurch gekennzeichnet, daß das Löseteil ein Langloch für die Aufnahme einer Halteschlaufe des Gurtes (15) aufweist.

7. Vorrichtung nach einem der Ansprüche 1 bis 6, dadurch gekennzeichnet, daß sich zwischen Stirnfläche (17) und Querrippe (12) auf der dem Klemmteil (1) gegenüberliegenden Seite des Gurtes (15) ein Freiraum befindet, der zumindest die Dicke des Gurtes hat oder größer als 2 mm ist.

## Claims

1. Device for stopping the flow of blood in extremities consisting of a flat strap (15) made of an elastic material that forms a loop (35) and of a strap-clamping device that is attached to the strap and that consists of a clamping part (1) and a release part (2); the clamping part has a foundation (29) that forms a base (39) for supporting and guiding the strap (15) in the longitudinal direction and that has side walls, which have first locking mechanisms for releasable fastening of the clamping part (1) and the release part (2), and between which the strap (15) passes; the release part (2) is attached to one end (22) of the strap, has second locking mechanisms for releasable fastening with the clamping part (1) and has a clamping zone (3) at the far end of the release part away from the loop (35); the distance of the clamping zone from the base (39) in the assembled state of the strap-locking device is approximately the same as the thickness of the loose strap (15); in the assembled state of the strap locking device, on the one hand, the first and second locking mechanisms connect the clamping part (1) with the release part (2), and on the other hand, the first and second locking mechanisms form a swivel joint; the release part (2) has an outer surface (18), which is located between the first and second locking mechanisms and the loop (35), and when the forward part of this outer surface (18) is pressed, the clamping zone (3) is separated from the base (39), thereby releasing the clamping action; the device is characterized by the fact that at the loop (35) end of the clamping part (1) a transverse rib (12) projects from the base (39) towards the release part (2), and that this transverse rib (12) projects from the base (39) by a distance at least equal to the thickness of the strap and preferably a distance equal to twice the thickness of the strap, so that the strap (15) runs down to the base (39) after passing the transverse rib (12).

2. Device in accordance with Claim 1, characterized by the fact that the transverse rib (12) forms an inclined surface (13) for supporting the strap and that the inclination of the inclined surface corresponds basically to the direction of the entering part of the loop (35) that rests upon it.

3. Device in accordance with Claim 1 or 2, characterized by the fact that the inclined surface (13) is inclined to the base (39) at an angle of 30 to 60° and preferably at an angle of 45°.

4. Device in accordance with any of Claims 1 to 3, characterized by the fact that the upper edge of the transverse rib (12) is inwardly displaced with respect to a front face (17) of the clamping part (1) facing the loop (35) and is preferably separated from the front face (17) by at least the width of the strap or by 4 to 6 mm.

5. Device in accordance with any of Claims 1 to 4, characterized by the fact that the strap is bent at the transverse rib (12) by at least 50° and preferably by at least 60°.

6. Device in accordance with any of Claims 1 to 5, characterized by the fact that the release part has a slot for holding a retaining loop of the strap (15).

7. Device in accordances with any of claims 1 to 6, characterized by the fact that a free space is provided between the front face (17) and the transverse rib (15) at the side of the strap (15) facing the clamping part (1), which free space at least has the thickness of the strap (15) or is larger than 2 mm.

## Revendications

1. Dispositif hémostatique applicable à des parties du corps avec une ceinture plate (15) sous la forme d'une courroie (35), en matériau élastique, sur laquelle est fixée une boucle constituée d'un élément de blocage (1) et d'un élément de desserrage (2); l'élément de blocage présente une partie de base (29) avec une surface (39) d'appui et de guidage de la ceinture (15) dans le sens longitudinal et qui comprend des parois latérales qui supportent le premier élément de verrouillage de la liaison amovible de l'élément de blocage (1) et de l'élément de desserrage (2), entre lesquels passe la ceinture (15) et où l'élément de desserrage (2) est relié à une extrémité de la ceinture (22), possède le deuxième élément de verrouillage pour la liaison amovible de l'élément de blocage (1) et présente à l'extrémité opposée à la boucle (35) une plage de blocage (3) dont l'intervalle par rapport à la surface (39), lorsque la boucle de la ceinture est fermée, se trouve dans la zone de l'épaisseur de la ceinture desserrée (15) et, lorsque la boucle de la ceinture est fermée, relie d'une part l'élément de serrage (1) à l'élément de desserrage (2) par le premier et le deuxième éléments de verrouillage et, d'autre part, le premier et deuxième éléments de verrouillage forment un joint articulé et, en cas de pression sur une surface extérieure avant (18) de l'élément de desserrage (2), surface qui se trouve entre le premier et le deuxième éléments de verrouillage et la boucle (35), la plage de blocage (3) est enlevée de la surface (39) et ainsi l'effet de blocage est supprimé, caractérisé par le fait que, sur l'extrémité de l'élément de serrage (1) dirigée vers la boucle (35), une nervure transversale (12) est en saillie de la surface (39) vers l'élément de desserrage (2), que cette nervure transversale (12) est en saillie de la surface (39) au moins de la simple épaisseur mais de préférence au moins du double de l'épaisseur de la ceinture, de façon que la ceinture (15) qui fait suite à la nervure transversale (12) passe sur la surface (39).

2. Dispositif selon la revendication 1, caractérisé par le fait que la nervure transversale (12) forme une surface oblique (13) pour le support de la ceinture dont le sens correspond essentiellement à celui de la partie rétrécie de la boucle (35) appliquée.

3. Dispositif selon la revendication 1 ou 2, caractérisé par le fait que la surface oblique (13) forme un angle de 30 à 60 degrés, de préférence de 45 degrés, par rapport à la surface (39).

4. Dispositif selon l'une des revendications 1 à 3, caractérisé par le fait que l'arête supérieure de la nervure transversale (12) est décalée vers l'intérieur par rapport à une face frontale (17) de l'élément de serrage (1) dirigée vers la boucle (35), et qu'elle soit de préférence éloignée au moins de la largeur de la ceinture ou de 4 à 6 mm de la face frontale (17).

5. Dispositif selon l'une des revendications 1 à 4, caractérisé par le fait que la ceinture, sur la nervure transversale (12), présente un repliement d'au moins 50 degrés, de préférence d'au moins 60 degrés.

6. Dispositif selon l'une des revendications 1 à 5, caractérisé par le fait que l'élément de desserrage comprend un trou oblong pour le logement d'une courroie de soutien de la ceinture (15).

7. Dispositif selon l'une des revendications 1 à 6, caractérisé par le fait qu'un espace libre au moins de l'épaisseur de la ceinture ou supérieur à 2 mm se trouve entre la surface frontale (17) et la nervure transversale (12) sur le côté de la ceinture (15) opposé à l'élément de serrage (1).
